# EUROPEAN PATENT SPECIFICATION

(11) **EP 3 307 174 B1**
(45) Date of publication and mention of the grant of the patent: **03.04.2019**
(21) Application number: 16728110.4
(22) Date of filing: 31.05.2016
(51) Int. Cl.: A61B 8/08, G01S 15/89, G01S 7/52

(54) **ULTRASONIC TRANSDUCER ARRAY PROBE FOR SHEAR WAVE IMAGING**
ULTRASCHALLWANDLERANORDNUNGSSONDE FÜR SCHERWELLENBILDGEBUNG
RÉSEAU DE TRANSDUCTEURS À ULTRASONS POUR SONDE D'IMAGERIE À ONDES DE CISAILLEMENT

(30) Priority: 11.06.2015 US 201562174091 P
(43) Date of publication of application: 18.04.2018
(73) Proprietor: Koninklijke Philips N.V., 5656 AE Eindhoven (NL)
(72) Inventor: OWEN, Neil, 5656 AE Eindhoven (NL); SHAMDASANI, Vijay Thakur, 5656 AE Eindhoven (NL); KUNKEL, Harry Amon, 5656 AE Eindhoven (NL); PETERS, Samuel Raymond, 5656 AE Eindhoven (NL)
(74) Representative: Mumbrú Forn, José
(86) International application number: PCT/IB2016/053186
(87) International publication number: WO 2016/198989

(56) References cited:
- WO-A1-2014/055973
- US-A1- 2010 069 751
- SONG PENGFEI ET AL: "Two-dimensional shear-wave elastography on conventional ultrasound scanners with time-aligned sequential tracking (TAST) and comb-push ultrasound shear elastography (CUSE)", IEEE TRANSACTIONS ON ULTRASONICS, FERROELECTRICS AND FREQUENCY CONTROL, IEEE, US, vol. 62, no. 2, 1 February 2015 (2015-02-01), pages 290-302, XP011571397, ISSN: 0885-3010, DOI: 10.1109/TUFFC.2014.006628 [retrieved on 2015-01-28]

## Description

This invention relates to medical diagnostic ultrasound systems and, in particular, to an ultrasonic transducer array probe for ultrasound systems which perform measurements of tissue stiffness or elasticity using shear waves.

One of the uses of diagnostic ultrasound is to diagnose lesions in the body by tissue elasticity or stiffness. For example, breast tumors or masses with high stiffness might be malignant, whereas softer and more compliant masses are likely to be benign. Since the stiffness of a mass is known to correlate with malignancy or benignity, the ultrasonic technique known as elastography can be used to provide the clinician with evidence to aid in diagnosis and determination of a treatment regimen.

Another approach to elasticity measurement is shear wave measurement. When a point on the body is compressed, then released, the underlying tissue is compressed downward, then rebounds back up when the compressive force is released. But since the tissue under the compressive force is continuously joined to surrounding tissue, the uncompressed tissue lateral of the force vector will respond to the up-and-down movement of the compressed tissue. A rippling effect in this lateral direction, referred to as a shear wave, is the response in the surrounding tissue to the downward compressive force. Furthermore, it has been determined that the force needed to push the tissue downward can be produced by the radiation pressure from an ultrasound pulse, commonly called a "push pulse." After the compressive force of the push pulse has ended and the tissue springs back, initiating the orthogonal shear wave, ultrasound reception can be used to sense and measure the tissue motion induced by the shear waves. Shear wave velocity is determined by local tissue mechanical properties. The shear wave will travel at one velocity through soft tissue, and at another, higher velocity through hard tissue. By measuring the velocity of the shear wave at a point in the body, information is obtained as to characteristics of the tissue such as its shear elasticity modulus, Young's modulus, and dynamic shear viscosity. The laterally propagating shear wave travels slowly, usually a few meters per second or less, making the shear wave susceptible to detection, although it attenuates rapidly over a few centimeters or less. See, for example, US Pat. 5,606,971 (Sarvazyan) and US Pat. 5,810,731 (Sarvazyan et al.) Since the same "push pulse" can be repeated for each measurement, the shear wave technique lends itself to objective quantification of tissue characteristics with ultrasound. Furthermore, the shear wave velocity is independent of the push pulse intensity, making the measurement less dependent upon the user.

But it requires time to acquire a series of data sets for shear wave analysis. As previously mentioned, the shear waves rapidly attenuate in the tissue. Furthermore, the tissue displacement caused by an ultrasonic push pulse is tiny, generally on the order of 30 micrometers or less. Consequently, it is usually necessary to repeat the shear wave measurement every few millimeters throughout the tissue being diagnosed. It would thus be desirable to be able to shorten the time required to make the necessary shear wave measurements throughout the tissue or organ being diagnosed, as by making several measurements simultaneously or generating shear waves of greater amplitude which can still be detected after passage through a greater tissue distance.

Document WO 2014/055973 A1 relates to a technique for performing shear wave elastography using push and/or detection ultrasound beams that are generated by subsets of the available number of transducer elements in an ultrasound transducer. This technique provides several advantages over currently available approaches to shear wave elastography, including the ability to use a standard, low frame rate ultrasound imaging system and the ability to measure shear wave speed throughout the entire field-of-view rather than only those regions where the push beams are not generated.

In some aspects, the present invention includes ultrasound systems. For example, the present invention includes an ultrasound system performing shear wave analysis. The ultrasound system can include a probe and a transmit beamformer with a given number of transmit channels. The system can also include an ultrasonic transducer array, located in the probe, and having a number of transducer elements that exceeds the given number. The system can include a switch multiplexer coupled between the transmit channels of the beamformer and the elements of the transducer array and can be configured to selectively couple each of the given number of transmit channels to transducer elements of a plurality of transmit apertures of a push pulse. The system can be configured to transmit a plurality of push pulses simultaneously when transmit channels of the beamformer are coupled to transducer elements of the plurality of transmit apertures.

In certain aspects, each transmit channel further includes a transmit signal source and an amplifier. The transmit signal source can include one of a pulser or a digital memory storing a transmit waveform in digital form. The switch matrix or multiplexer can include a plurality of single pole, single throw switches. The system can be configured to selectively connect each transmit channel by the closure of one or more of the plurality of switches to the at least one transducer

In some embodiments, a size of one of the transmit apertures is equal to a given number of transducer elements. The number of channels of the transmit beamformer can be 128 and a size of one of the transmit apertures is equal to 128 transducer elements. The system can also include a probe cable having signal lines coupling the transmit channels of the transmit beamformer to the switch multiplexer. The probe can include a probe handle and a distal end, wherein the switch multiplexer is located in the probe handle and the transducer array is located in the distal end. Alternatively, the probe can include a probe handle and a distal end, wherein the switch multiplexer and the transducer array are located in the distal end. In some aspects, the system can include a probe connector and a probe cable having signal lines coupling the probe connector to the transducer array, wherein the switch multiplexer is located in the probe connector.

In some aspects, the present invention can include methods for operating an ultrasound system. For example, the present invention can include a method for operating an ultrasound system to measure shear waves, the ultrasound system having a given number of transmit channels each having a signal source, and an ultrasonic array transducer having a number of transducer elements which is greater than the given number and a switch multiplexer of switches coupling the transmit channels to the transducer elements. The method can include closing switches of the switch multiplexer to couple transmit channels to transducer elements of more than one push pulse transmit aperture, wherein each of a plurality of individual channels is coupled to the transducer elements of a plurality of push pulse transmit apertures, and actuating the signal sources of the transmit channels to simultaneously transmit a plurality of push pulses from the array transducer. In certain aspects, the actuating can include actuating the signal sources of the transmit channels to simultaneously transmit a plurality of identical push pulses in parallel from the ultrasonic array transducer. The number of transducer elements of two apertures of the plurality of push pulse transmit apertures can be greater than the given number of transmit channels. The methods can also include repeating the closing and actuating steps with the closed switches changed to couple the transmit channels to transducer elements of different push pulse transmit apertures in the plurality.

In the drawings:
FIGURE 1 illustrates in block diagram form an ultrasonic diagnostic imaging system which performs shear wave imaging with a probe of the present invention.
FIGURE 2 is a schematic illustration of channels of a transmit beamformer coupled by a switch matrix or multiplexer to the elements of a transducer array in accordance with the principles of the present invention.
FIGURE 3 illustrates possible locations for a switch matrix or multiplexer in a probe of the present invention, including the probe connector and the probe handle.

In some embodiments, the present invention includes an ultrasonic array transducer probe is described which is capable of transmitting multiple simultaneous push pulses for shear wave imaging. The probe is operated by switches of a switch matrix or multiplexer which can be set to couple individual channels of an ultrasound system transmit beamformer to multiple elements of different transmit apertures of the array transducer. The transmit beamformer can thereby transmit multiple, laterally separate push pulses from different probe apertures at the same time, causing multiple shear waves to be generated for interrogation at the same time or the development of constructive interference in the form of a stronger shear wave amplitude in the body.

The subject of the present invention is an ultrasound probe suitable for use in shear wave imaging procedures to transmit multiple simultaneous push pulses for the stimulation of shear waves. A preferred probe is designed for use with a standard ultrasound system beamformer which may have fewer transmit channels than the number of elements of the probe's transducer array. For instance, this permits a probe with a transducer array of more than 128 elements to be used with a standard transmit beamformer of 128 channels. This is accomplished by a switch matrix or switch multiplexer that selectively connects channels of the transmit beamformer to transducer elements of multiple apertures so that the transmission will result in multiple push pulses being transmitted from the multiple transducer apertures. Probes have been used with multiplexers in the past to selectively connect beamformer channels to elements of the array transducer. A familiar example is the switching of the active aperture along the array of a linear array probe, an operation commonly referred to as "tractor-treading." For instance, eight channels of a beamformer may be translated from one end of a 128-element array to the other to transmit and receive a beam at each position along the array. Both the transmit and receive apertures are switched along the array, and the switching is conventionally done in the system beamformer, not the probe itself. Only one beam is sent and received at a time. US Pat. 8,161,817 (Robinson et al.) illustrates tractor-treading of received signals to the receive beamformer by a probe microbeamformer in a two-dimensional array transducer. Another well-known use of switching a probe is known as synthetic aperture, commonly done when there are fewer receive beamformer channels than there are transducer elements. In this technique, transmission is done twice with the full transducer aperture, and reception is done of different halves of the aperture each time. The received half-apertures are then combined to form the full aperture, as described, for instance, in US Pat. 6,050,942 (Rust et al.) There must be sufficient transmit channels to transmit over the full aperture each time, however. Another technique in which fewer beamformer channels than array elements are used is known as a folded aperture, which takes advantage of aperture symmetry to send and receive signals on pairs of transducer elements. For instance, consider a five-element aperture of elements 1-5, with element #3 being the center element. The elements can be paired so that elements #1 and #5 are connected to a single beamformer channel on receive, as are elements #2 and #4, with center element #3 connected to its own channel for beamforming. See, for example, US Pat. 5,893,363 (Little et al.) The same pairing can be done on transmit. Folded apertures however can only be used to steer beams straight ahead; when beams are steered from side to side the symmetrically-located elements require different delays and pairing cannot be done.

The foregoing examples are mainly of element and channel switching during reception and all transmission and reception being of only one beam at a time. The reason for this is that ultrasound is used principally for imaging and the use of multiple transmit beams during imaging will cause image degradation known as clutter. On reception, the signals received for a receive beam from one transmit beam will be contaminated with echoes received from the other transmit beam which will appear in the reconstructed image as clutter. A number of proposals have been put forward for multiple beam transmission because it should decrease the time to scan the image field and hence increase the frame rate of display. US Pat. 7,537,567 (Jago et al.) is one such proposal, which attempts to reduce the clutter by transmitting the multiple simultaneous imaging beams in sharply divergent directions. The inventors recognize the clutter problem as shown by the several precautions they recommend at the end of the patent to minimize the problem. The present inventors have taken advantage of the fact that shear wave imaging is not conventional pulse-echo imaging, but has as its purpose the measurement of a laterally propagating shear wave resulting from a push pulse. The echoes returned from the push pulse transmission itself are not used for anatomical imaging and consequently image clutter is not an issue.

The question arises in designing an implementation of the present invention of where to locate the switches of the matrix or multiplexer. In linear array imaging as described above, the switches for aperture switching are in the system mainframe, generally at the output of the beamformer. An implementation of the present invention can locate the switches in the system mainframe if desired, but this would create in most instances the need for a non-standard system beamformer and transducer socket. One desire of an implementation of the present invention is that it be used with a standard ultrasound system with standard probe sockets. This leads to a second possible location, the probe connector at the end of the probe cable which connects the probe to the system mainframe. It is known, for instance, to locate the amplifier for a therapy probe in the probe connector as shown in US Pat. pub. no. 2008/0228075 (Fraser et al.), and also to locate memory devices there which inform the ultrasound system of performance characteristics of the probe as described in US Pat. 4,868,476 (Respaut). However, locating the switches in the probe connector will cause the cable to have a large number of signal conductors, one for each element of the array, undesirably increasing the size and weight of the probe cable. Hence the preferred location for the switches is in the probe case itself, which enables both a lightweight probe cable and use of the probe with a standard ultrasound system.

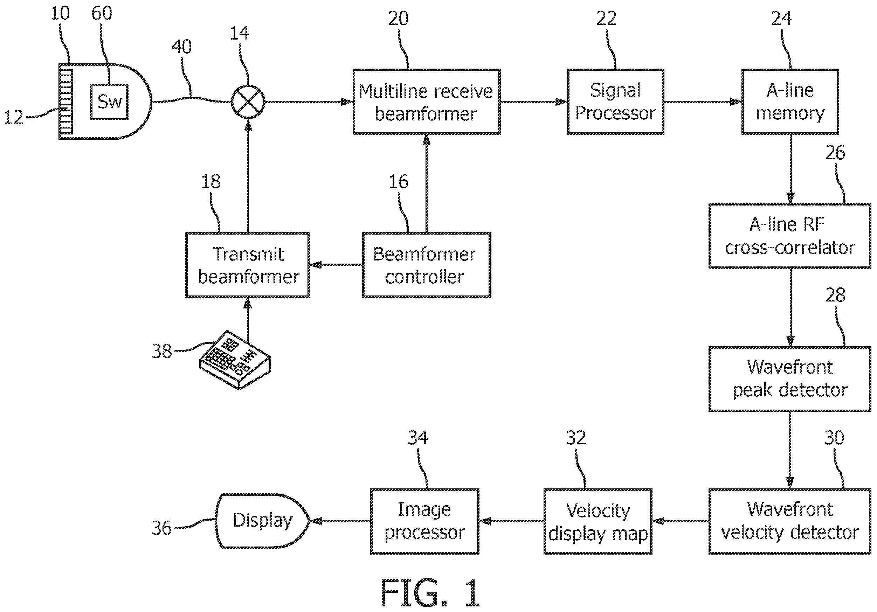

Referring now to FIGURE 1, an ultrasound system designed for the measurement of shear waves and used with an ultrasound probe of the present invention is shown in block diagram form. The ultrasound probe 10 has a transducer array 12 of transducer elements which operate to transmit and receive ultrasound signals. The array can be a one dimensional (1D) or a two dimensional (2D) array of transducer elements. In a constructed embodiment the array transducer is a so-called 1.25D array having a central azimuthal row of elements flanked by a few parallel rows to provide limited focusing in the elevation direction. Either type of array can scan a 2D plane and the two dimensional array can be used to scan a volumetric region in front of the array. A probe cable 40 connects the probe to the ultrasound system mainframe. The array elements are coupled to a transmit beamformer 18 and a multiline receive beamformer 20 in the ultrasound system by a transmit/receive (T/R) switch 14. Transmit beamformers are well known in the art and are described in US Pat. pub. no. 2013/0131511 (Peterson et al.), US Pat. 6,937,176 (Freeman et al.), US Pat. 7,715,204 (Miller), and US Pat. 5,581,517 (Gee et al.) for instance. As described in these publications, a transmit beamformer for an array transducer has multiple channels, each of which can transmit a drive pulse or waveform at an independently programmed time in relation to the other channels. It is the selected relative timing of the application of the drive signals to the individual transducer elements which provides transmit beam focusing and steering. Coordination of transmission and reception by the beamformers is controlled by a beamformer controller 16, which is controlled by user operation of a user control panel 38. The user can operate the control panel to command the ultrasound system to transmit a single push pulse or multiple simultaneous push pulses during shear wave imaging, for instance. The multiline receive beamformer produces multiple, spatially distinct receive lines (A-lines) of echo signals during a single transmit-receive interval. Multiline beamformers are known in the art as described in US Pat. 6,482,157 (Robinson), US Pat. 6,695,783 (Henderson et al.), and US Pat. 8,137,272 (Cooley et al.), for instance. The echo signals are processed by filtering, noise reduction, and the like by a signal processor 22, then stored in an A-line memory 24, a digital memory which stores the echo signal data received along the A-lines. Temporally distinct A-line samples relating to the same spatial vector location are associated with each other in an ensemble of echoes relating to a common point in the image field. The r.f. echo signals of successive A-line sampling of the same spatial vector are cross-correlated by an A-line r.f. cross-correlator 26, a processor programmed to perform cross-correlation of signal data, to produce a sequence of samples of tissue displacement for each sampling point on the vector. Alternatively, the A-lines of a spatial vector can be Doppler processed to detect shear wave motion along the vector, or another phase-sensitive techniques can be employed. A wavefront peak detector 28 is responsive to detection of the shear wave displacement along the A-line vector to detect the peak of the shear wave displacement at each sampling point on the A-line. In a preferred embodiment this is done by a processor performing curve-fitting, although cross-correlation and other interpolative techniques can also be employed if desired. The time at which the peak of the shear wave displacement occurs is noted in relation to the times of the same event at other A-line locations, all to a common time reference, and this information is coupled to a wavefront velocity detector 30, a processor which differentially calculates the shear wave velocity from the peak displacement times on adjacent A-lines. This velocity information is coupled into a velocity display map 32 stored in a buffer, which indicates the velocity of the shear wave at spatially different points in a 2D or 3D image field. The velocity display map is coupled to an image processor 34 which processes the velocity map, preferably overlaying an anatomical ultrasound image of the tissue, for display on an image display 36. Further details of the ultrasound system components of FIGURE 1 can be found in US Pat. pub. no. 2013/0131511 (Peterson et al.)

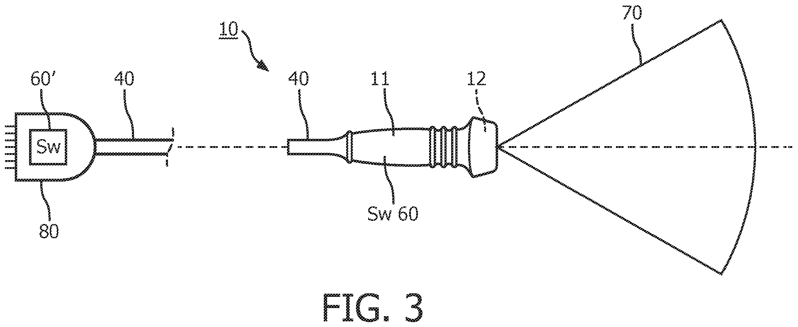

FIGURE 3 is an illustration of a probe 10 of the present invention which shows two of the possible locations for a switch matrix or multiplexer described above. At the left side of the drawing is a probe connector 80, which is seen to be connected to the probe 10 by the probe cable 40. A typical probe cable can be upwards of two meters in length. The switch matrix or multiplexer 60' can be located in the probe connector 80 and coupled to the transducer array 12 in the probe by the cable 40. However, as mentioned above, this would undesirably increase the number of signal conductors in the cable and hence the size and weight of the cable, as there would need to be a signal conductor for every element of the array. The preferred location for the switch matrix or multiplexer is in the handle 11 of the probe 10 as indicated by Sw. 60 and also shown in FIGURE 1. If a microbeamformer is used in the probe, it is also possible to implement the switch matrix or multiplexer in solid state form as part of the microbeamformer, just behind the array transducer 12 in the distal end of the probe. This drawing also shows a typical image (scanning) field 70 in front of the distal end of the probe. The image field can also be rectilinear in shape when linear array scanning is used.

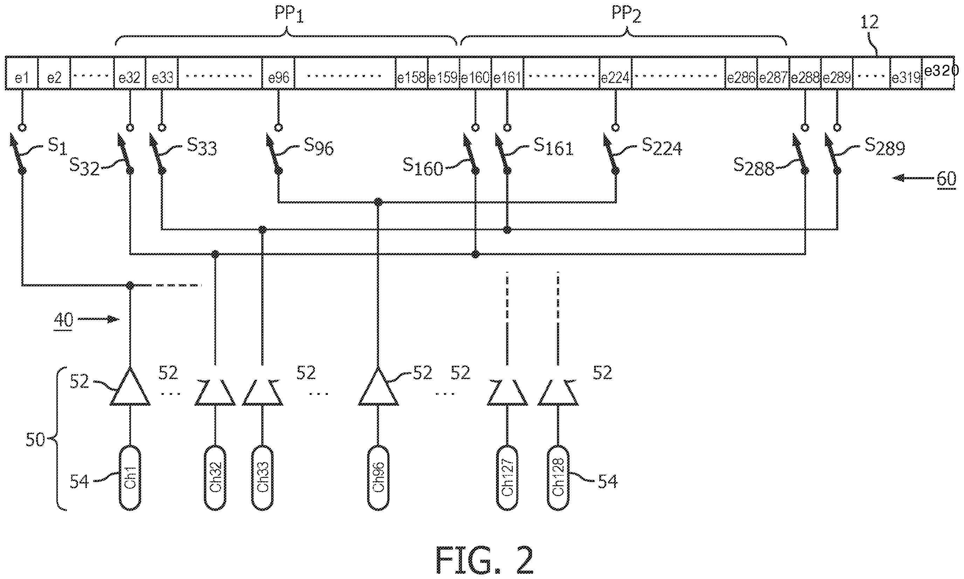

FIGURE 2 is a schematic illustration of an array transducer probe constructed in accordance with the principles of the present invention. In this example the array transducer 12 has 320 elements, labeled e1 to e320. The transmit beamformer is a 128-channel beamformer, with the channels 50 shown at the bottom of the drawing. Each beamformer transmit channel has a transmit signal source 54, which may be a pulser such as shown in US Pat. pub. no. 2011/0237953 (Olsson et al.) or US Pat. 6,540,682 (Leavitt et al.), or a digital memory storing a transmit waveform in digital form. In the latter case, at the time of transmission the digital waveform is clocked out of the memory and converted to an analog waveform by an A/D converter. See US Pat. 5,581,517 (Gee et al.) for an example of this form of transmit beamformer. For a push pulse, pulses of high MI and long durations are used so that sufficient energy is transmitted to displace the tissue downward along the beam direction and cause the development of a shear wave. In some embodiments, pulses from 50 to 1000 microseconds can be used. For example, each push pulse can be a long pulse of 50 to 200 microseconds in duration. One example duration is 100 microseconds. In certain embodiments, longer pulses ranging from 400 to 1000 microseconds can be used. The transmit pulse or waveform is then amplified by an amplifier 52 and coupled to a transducer element. In this example the transmit waveforms are coupled over signal lines of the probe cable 40 to a switch matrix or multiplexer 60 in the probe. In this implementation each beamformer channel is coupled by single pole, single throw switches Sₙ to one, two or three transducer elements eN. Each amplifier 52 is thus of sufficient output power to drive the impedances of one, two or three transducer elements in parallel. Channel 1, for example, is coupled by switch S₁ to the first element e1, and also by other switches (not shown) to elements e129 and e257, an element spacing equal to the channel count of 128. Other connection sets are similarly spaced. The illustrated arrangement shows some of the switches for the transmission of two simultaneous push pulses PP₁ and PP₂ from one 128-element aperture of e23 to e159 and another 128-element aperture of e160 to e287. Channel 32 is coupled by S₃₂ to element e32 and by S₁₆₀ to element e160. During transmission both of these switches are closed to drive the left-most element of each aperture in parallel. Channel 32 is also coupled to element S₂₈₈ by switch S₂₈₈, but this switch remains open in this exemplary aperture configuration. Similarly, channel 33 is coupled by switches S₃₃ and S₁₆₁ to drive elements e33 and e161 of the two apertures, while switch S₂₈₉ of channel 33 remains open. Each aperture has a central element, e96 and e224, which marks the center axis of the respective push pulse and is driven from channel 96 by the closure of switches S₉₆ and S₂₂₄, respectively. Channel 96 is one of the channels which is only coupled to two transducer elements. In operation, the signal source 54 of each channel is actuated at the appropriate time for the transmitted push pulse to be steered and focused in the desired direction and at the desired depth. Identical push pulses PP₁ and PP₂ are thus transmitted simultaneously and in parallel to stimulate one or two shear waves. In a typical implementation with typical element pitch, the distance between the center axes of 128-element apertures would be about 20mm. By setting other switch combinations and/or using other aperture sizes, the push pulse axes can be shifted along the array and transmitted from other aperture locations and with other spacings. Single or multiple push pulses can be transmitted as desired, and successive push pulses can be applied as described in the aforementioned US Pat. pub. no. 2013/0131511 (Peterson et al.)

Following push pulse transmission, the switches of the matrix or multiplexer 60 are opened and the array 12 is operated to sample and measure the resultant shear wave. When the system of FIGURE 1 is used, focused tracking pulses are transmitted and resultant echoes received by the probe 10 in the vicinity of the push pulse which generates the shear wave. A typical transmit tracking pulse is a short pulse, usually only one or two cycles, at a frequency suitable for penetrating the depth being studied, such as 7-8 MHz. Each tracking pulse vector is repetitively sampled in a time-interleaved manner so that tissue motion produced by a shear wave can be detected when it occurs at each tracking pulse vector location, preferably by correlating the echo data from successive interrogations of the vector. As the shear wave moves laterally away from the push pulse axis, the positioning of the tracking pulses can also be moved laterally to follow the propagation of the shear wave. The data from the repetitively sampled tracking pulse vectors is processed to find the times at which the shear wave causes a peak displacement at each point of the tracking pulse vector, preferably by cross-correlation, curve-fitting or interpolating successive displacement measurements. Analysis of the times at which points on adjacent sampling vectors experience peak displacement produces a measurement corresponding to the velocity of the shear wave at particular vector locations, with velocity variations indicating tissues of different stiffness or elasticity. Since the shear waves attenuate so rapidly, it is generally not possible to acquire shear wave data from an entire image field 70 with a single push pulse vector. Thus, the process is repeated at other locations in the tissue to acquire shear wave velocity measurements in other region of the tissue. A probe of the present invention can shorten the time required to do this by enabling multiple locations to be measured at the same time or stronger shear waves to be generated. The process is repeated until shear wave data has been acquired over the desired image field. The velocity information is preferably presented as a two- or three-dimensional image of the tissue, color-coded by the shear wave velocity data at points in the image.

It should be noted that the various embodiments described above and illustrated, e.g., by the exemplary ultrasound system of FIGURE 1 may be implemented in hardware, software or a combination thereof. The various embodiments and/or components, for example, the modules, or components and controllers therein, also may be implemented as part of one or more computers or microprocessors. The computer or processor may include a computing device, an input device, a display unit and an interface, for example, for accessing the Internet. The computer or processor may include a microprocessor. The microprocessor may be connected to a communication bus, for example, to access a PACS system. The computer or processor may also include a memory. The memory may include Random Access Memory (RAM) and Read Only Memory (ROM). The computer or processor further may include a storage device, which may be a hard disk drive or a removable storage drive such as a floppy disk drive, optical disk drive, solid-state thumb drive, and the like. The storage device may also be other similar means for loading computer programs or other instructions into the computer or processor.

As used herein, the term "computer" or "module" or "processor" may include any processor-based or microprocessor-based system including systems using microcontrollers, reduced instruction set computers (RISC), ASICs, logic circuits, and any other circuit or processor capable of executing the functions described herein. The above examples are exemplary only, and are thus not intended to limit in any way the definition and/or meaning of these terms.

The computer or processor executes a set of instructions that are stored in one or more storage elements, in order to process input data. The storage elements may also store data or other information as desired or needed. The storage element may be in the form of an information source or a physical memory element within a processing machine.

The set of instructions may include various commands that instruct the computer or processor as a processing machine to perform specific operations such as the methods and processes of the various embodiments of the invention. The set of instructions may be in the form of a software program. The software may be in various forms such as system software or application software and which may be embodied as a tangible and non-transitory computer readable medium. Further, the software may be in the form of a collection of separate programs or modules, a program module within a larger program or a portion of a program module. The software also may include modular programming in the form of object-oriented programming. The processing of input data by the processing machine may be in response to operator commands, or in response to results of previous processing, or in response to a request made by another processing machine.

## Claims

1. An ultrasound system performing shear wave analysis, the ultrasound system comprising a probe (10) and having a transmit beamformer (18) with a given number of transmit channels (50), the system comprising:
an ultrasonic transducer array (12), located in the probe, and having a number of transducer elements (e1-e320),
wherein the system is configured to transmit a plurality of push pulses (PP1, PP2) simultaneously when transmit channels (50) of the beamformer are coupled to transducer elements (e1-e320),
**characterized in that** the number of transducer elements (e1-e320) exceeds the given number of transmit channels (50),
**in that** the system further comprises a switch multiplexer (60) coupled between the transmit channels (50) of the beamformer and the elements (e1-e320) of the transducer array and configured to selectively couple each of the given number of transmit channels to transducer elements of a plurality of transmit apertures of a push pulse, and
**in that** the system is configured to transmit a plurality of push pulses (PP1, PP2) simultaneously when transmit channels (50) of the beamformer are coupled to transducer elements (e1-e320) of the plurality of transmit apertures.

2. The ultrasound system of Claim 1, wherein each transmit channel (50) further comprises a transmit signal source (54) and an amplifier (52).

3. The ultrasound system of Claim 2, wherein the transmit signal source (54) comprises one of a pulser or a digital memory storing a transmit waveform in digital form.

4. The ultrasound system of Claim 1, wherein the switch multiplexer (60) comprises a plurality of single pole, single throw switches (S₁-S₂₈₉).

5. The ultrasound system of Claim 4, wherein the system is configured to selectively connect each transmit channel (50) by the closure of one or more of the plurality of switches (S₁-S₂₈₉) to the at least one transducer element (e1-e320).

6. The ultrasound system of Claim 1, wherein a size of one of the transmit apertures is equal to a given number of transducer elements (e1-e320).

7. The ultrasound system of Claim 1, wherein the number of channels (50) of the transmit beamformer is 128 and a size of one of the transmit apertures is equal to 128 transducer elements (e1-e320).

8. The ultrasound system of Claim 1, further comprising a probe cable (40) having signal lines coupling the transmit channels (50) of the transmit beamformer to the switch multiplexer (60).

9. The ultrasound system of Claim 8, wherein the probe (10) further comprises a probe handle (11) and a distal end,
wherein the switch multiplexer (60) is located in the probe handle and the transducer array (12) is located in the distal end.

10. The ultrasound system of Claim 8, wherein the probe (10) further comprises a probe handle (11) and a distal end,
wherein the switch multiplexer (60) and the transducer array (12) are located in the distal end.

11. The ultrasound system of Claim 1, further comprising a probe connector (80) and a probe cable (40) having signal lines coupling the probe connector to the transducer array (12),
wherein the switch multiplexer (60') is located in the probe connector.

12. A method for operating an ultrasound system to measure shear waves, the ultrasound system having a given number of transmit channels (50) each having a signal source, and an ultrasonic array transducer (12) having a number of transducer elements (e1-e320) which is greater than the given number and a switch multiplexer (60) of switches coupling the transmit channels to the transducer elements, the method comprising:
closing switches (S₁-S₂₈₉) of the switch multiplexer to couple transmit channels (50) to transducer elements (e1-e320) of more than one push pulse transmit aperture, wherein each of a plurality of individual channels is coupled to the transducer elements of a plurality of push pulse transmit apertures; and
actuating the signal sources (54) of the transmit channels to simultaneously transmit a plurality of push pulses (PP1, PP2) from the array transducer (12).

13. The method of Claim 12, wherein the actuating comprises actuating the signal sources (54) of the transmit channels to simultaneously transmit a plurality of identical push pulses (PP1, PP2) in parallel from the ultrasonic array transducer (12).

14. The method of Claim 12, wherein the number of transducer elements (e1-e320) of two apertures of the plurality of push pulse transmit apertures is greater than the given number of transmit channels (50).

15. The method of Claim 12, further comprising repeating the closing and actuating steps with the closed switches changed to couple the transmit channels (50) to transducer elements (e1-e320) of different push pulse transmit apertures in the plurality.

## Patentansprüche

1. Ultraschallsystem, das Scherwellenanalyse durchführt, wobei das Ultraschallsystem eine Sonde (10) umfasst und einen Sendestrahlformer (18) mit einer gegebenen Anzahl an Sendekanälen (50) aufweist, wobei das System umfasst:
ein Ultraschall-Wandlerarray (12), das in der Sonde lokalisiert ist und eine Anzahl an Wandlerelementen (e1-e320) aufweist,
wobei das System dazu konfiguriert ist, eine Vielzahl von Druckpulsen (PP1, PP2) gleichzeitig zu senden, wenn Sendekanäle (50) des Strahlformers mit Wandlerelementen (e1-e320) gekoppelt sind,
**dadurch gekennzeichnet, dass** die Anzahl an Wandlerelementen (e1-e320) die gegebene Anzahl an Sendekanälen (50) übersteigt,
dadurch, dass das System weiter einen Schaltmultiplexer (60) umfasst, der zwischen die Sendekanäle (50) des Strahlformers und die Elemente (e1-e320) des Wandlerarrays gekoppelt und dazu konfiguriert ist, selektiv jeden aus der gegebenen Anzahl an Sendekanälen mit Wandlerelementen aus einer Vielzahl von Sendeaperturen eines Druckpulses zu koppeln, und
dadurch, dass das System dazu konfiguriert ist, eine Vielzahl von Druckpulsen (PP1, PP2) gleichzeitig zu senden, wenn Sendekanäle (50) des Strahlformers mit Wandlerelementen (e1-e320) aus der Vielzahl von Sendeaperturen gekoppelt sind.

2. Ultraschallsystem nach Anspruch 1, wobei jeder Sendekanal (50) weiter eine Sendesignalquelle (54) und einen Verstärker (52) umfasst.

3. Ultraschallsystem nach Anspruch 2, wobei die Sendesignalquelle (54) eines aus einem Pulsgeber oder einem digitalen Speicher umfasst, der eine Sendewellenform in digitaler Form speichert.

4. Ultraschallsystem nach Anspruch 1, wobei der Schaltmultiplexer (60) eine Vielzahl von einpoligen Ein-/Ausschaltern (S₁-S₂₈₉) umfasst.

5. Ultraschallsystem nach Anspruch 4, wobei das System dazu konfiguriert ist, selektiv jeden Sendekanal (50) durch das Schließen von einem oder mehreren aus der Vielzahl von Schaltern (S₁-S₂₈₉) mit dem mindestens einen Wandlerelement (e1-e320) zu verbinden.

6. Ultraschallsystem nach Anspruch 1, wobei eine Größe einer der Sendeaperturen gleich einer gegebenen Anzahl an Wandlerelementen (e1-e320) ist.

7. Ultraschallsystem nach Anspruch 1, wobei die Anzahl an Kanälen (50) des Sendestrahlformers 128 beträgt und eine Größe einer der Sendeaperturen gleich 128 Wandlerelementen (e1-e320) ist.

8. Ultraschallsystem nach Anspruch 1, weiter ein Sondenkabel (40) umfassend, das Signalleitungen aufweist, die die Sendekanäle (50) des Sendestrahlformers mit dem Schaltmultiplexer (60) koppeln.

9. Ultraschallsystem nach Anspruch 8, wobei die Sonde (10) weiter einen Sondengriff (11) und ein distales Ende umfasst,
wobei der Schaltmultiplexer (60) im Sondengriff lokalisiert ist und das Wandlerarray (12) im distalen Ende lokalisiert ist.

10. Ultraschallsystem nach Anspruch 8, wobei die Sonde (10) weiter einen Sondengriff (11) und ein distales Ende umfasst,
wobei der Schaltmultiplexer (60) und das Wandlerarray (12) im distalen Ende lokalisiert sind.

11. Ultraschallsystem nach Anspruch 1, weiter einen Sondenstecker (80) und ein Sondenkabel (40) umfassend, welches Signalleitungen aufweist, die den Sondenstecker mit dem Wandlerarray (12) koppeln,
wobei der Schaltmultiplexer (60') im Sondenstecker lokalisiert ist.

12. Verfahren zum Betreiben eines Ultraschallsystems, um Scherwellen zu messen, wobei das Ultraschallsystem eine gegebene Anzahl an Sendekanälen (50) aufweist, die jeder eine Signalquelle aufweisen, und einen Ultraschall-Arraywandler (12), der eine Anzahl an Wandlerelementen (e1-e320) aufweist, die größer ist als die gegebene Anzahl, und einen Schaltmultiplexer (60) von Schaltern, welche die Sendekanäle mit den Wandlerelementen koppeln, wobei das Verfahren umfasst:
Schließen von Schaltern (S₁-S₂₈₉) des Schaltmultiplexers, um Sendekanäle (50) mit Wandlerelementen (e1-e320) von mehr als einer Druckpuls-Sendeapertur zu koppeln, wobei jeder aus einer Vielzahl von Einzelkanälen mit den Wandlerelementen einer Vielzahl von Druckpuls-Sendeaperturen gekoppelt wird; und
Betätigen der Signalquellen (54) der Sendekanäle, um gleichzeitig eine Vielzahl von Druckpulsen (PP1, PP2) aus dem Arraywandler (12) zu senden.

13. Verfahren nach Anspruch 12, wobei das Betätigen das Betätigen der Signalquellen (54) der Sendekanäle umfasst, um gleichzeitig eine Vielzahl von identischen Druckpulsen (PP1, PP2) parallel aus dem Ultraschall-Arraywandler (12) zu senden.

14. Verfahren nach Anspruch 12, wobei die Anzahl an Wandlerelementen (e1-e320) von zwei Aperturen aus der Vielzahl von Druckpuls-Sendeaperturen größer ist als die gegebene Anzahl an Sendekanälen (50).

15. Verfahren nach Anspruch 12, weiter das Wiederholen der Schließ- und Betätigungsschritte mit den geschlossenen Schaltern so geändert umfassend, dass die Sendekanäle (50) mit Wandlerelementen (e1-e320) von anderen Druckpuls-Sendeaperturen in der Vielzahl gekoppelt werden.

## Revendications

1. Système ultrasonore effectuant une analyse d'ondes de cisaillement, le système ultrasonore comprenant une sonde (10) et ayant un formateur de faisceaux de transmission (18) avec un nombre donné de canaux de transmission (50), le système comprenant :
un réseau de transducteurs ultrasoniques (12) situés dans la sonde et ayant un nombre d'éléments transducteurs (e1-e320),
dans lequel le système est configuré pour transmettre une pluralité d'impulsions de poussée (PP1, PP2) simultanément lorsque des canaux de transmission (50) du formateur de faisceaux sont couplés à des éléments transducteurs (e1-e320),
**caractérisé en ce que** le nombre d'éléments transducteurs (e1-e320) dépasse le nombre donné de canaux de transmission (50),
**en ce que** le système comprend en outre un multiplexeur de commutateurs (60) couplé entre les canaux de transmission (50) du formateur de faisceaux et les éléments (e1-e320) du réseau de transducteurs et configurés pour coupler sélectivement chacun du nombre donné de canaux de transmission aux éléments transducteurs d'une pluralité d'ouvertures de transmission d'une impulsion de poussée et
**en ce que** le système est configuré pour transmettre une pluralité d'impulsions de poussée (PP1, PP2) simultanément lorsque des canaux de transmission (50) du formateur de faisceaux sont couplés à des éléments transducteurs (e1-e320) de la pluralité d'ouvertures de transmission.

2. Système ultrasonore selon la revendication 1, dans lequel chaque canal de transmission (50) comprend en outre une source de signal de transmission (54) et un amplificateur (52).

3. Système ultrasonore selon la revendication 2, dans lequel la source de signal de transmission (54) comprend l'un(e) d'un générateur d'impulsions ou d'une mémoire numérique stockant une forme d'onde de transmission sous forme numérique.

4. Système ultrasonore selon la revendication 1, dans lequel le multiplexeur de commutateurs (60) comprend une pluralité de commutateurs unipolaires unidirectionnels (S₁-S₂₈₉).

5. Système ultrasonore selon la revendication 4, dans lequel le système est configuré pour connecter sélectivement chaque canal de transmission (50) par la fermeture d'un ou plusieurs de la pluralité de commutateurs (S₁-S₂₈₉) à au moins un élément transducteur (e1-e320).

6. Système ultrasonore selon la revendication 1, dans lequel une taille de l'une des ouvertures de transmission est égale à un nombre donné d'éléments transducteurs (e1-e320).

7. Système ultrasonore selon la revendication 1, dans lequel le nombre de canaux (50) du formateur de faisceaux de transmission est de 128 et une taille de l'une des ouvertures de transmission est égale à 128 éléments transducteurs (e1-e320).

8. Système ultrasonore selon la revendication 1, comprenant en outre un câble de sonde (40) ayant des lignes de signalisation couplant les canaux de transmission (50) du formateur de faisceaux de transmission au multiplexeur de commutateurs (60).

9. Système ultrasonore selon la revendication 8, dans lequel la sonde (10) comprend en outre une poignée de sonde (11) et une extrémité distale,
dans lequel le multiplexeur de commutateurs (60) est situé dans la poignée de sonde et le réseau de transducteurs (12) est situé dans l'extrémité distale.

10. Système ultrasonore selon la revendication 8, dans lequel la sonde (10) comprend en outre une poignée de sonde (11) et une extrémité distale,
dans lequel le multiplexeur de commutateurs (60) et le réseau de transducteurs (12) sont situés dans l'extrémité distale.

11. Système ultrasonore selon la revendication 1, comprenant en outre un connecteur de sonde (80) et un câble de sonde (40) ayant des lignes de signalisation couplant le connecteur de sonde au réseau de transducteurs (12),
dans lequel le multiplexeur de commutateurs (60') est situé dans le connecteur de sonde.

12. Procédé de fonctionnement d'un système ultrasonore pour mesurer des ondes de cisaillement, le système ultrasonore ayant un nombre donné de canaux de transmission (50) ayant chacun une source de signal et un transducteur de réseau ultrasonique (12) ayant un nombre d'éléments transducteurs (e1-e320) qui est supérieur au nombre donné et un multiplexeur de commutateurs (60)couplant les canaux de transmission aux éléments transducteurs, le procédé comprenant :
la fermeture de commutateurs (S₁-S₂₈₉) du multiplexeur de commutateurs pour coupler des canaux de transmission (50) à des éléments transducteurs (el-e320) de plus d'une ouverture de transmission d'impulsions de poussée, dans lequel chacun d'une pluralité de canaux individuels est couplé aux éléments transducteurs d'une pluralité d'ouvertures de transmission d'impulsions de poussée ; et
l'actionnement des sources de signal (54) des canaux de transmission pour transmettre simultanément une pluralité d'impulsions de poussée (PP1, PP2) depuis le transducteur de réseau (12).

13. Procédé selon la revendication 12, dans lequel l'actionnement comprend l'actionnement des sources de signal (54) des canaux de transmission pour transmettre simultanément une pluralité d'impulsions de poussée identiques (PP1, PP2) en parallèle du transducteur de réseau ultrasonique (12).

14. Système ultrasonore selon la revendication 12, dans lequel le nombre d'éléments transducteurs (e1-e320) de deux ouvertures de la pluralité d'ouvertures de transmission d'impulsions de poussée est supérieur au nombre donné de canaux de transmission (50).

15. Système ultrasonore selon la revendication 12, comprenant en outre la répétition des étapes de fermeture et d'actionnement avec les commutateurs fermés changés pour coupler les canaux de transmission (50) à des éléments transducteurs (e1-e320) de différentes ouvertures de transmission d'impulsions de poussée de la pluralité.
